(19) Europäisches Patentamt
European Patent Office
Office européen des brevets

(11) **EP 3 183 219 B1**

(12) **EUROPÄISCHE PATENTSCHRIFT**

(45) Veröffentlichungstag und Bekanntmachung des Hinweises auf die Patenterteilung:
**10.06.2020  Patentblatt 2020/24**

(21) Anmeldenummer: **15756601.9**

(22) Anmeldetag: **20.08.2015**

(51) Int Cl.:
***C02F 3/30*** (2006.01)

(86) Internationale Anmeldenummer:
**PCT/EP2015/069189**

(87) Internationale Veröffentlichungsnummer:
**WO 2016/026945 (25.02.2016 Gazette 2016/08)**

(54) **VERFAHREN ZUR ERMITTLUNG DER MENGE AN ANAMMOX-BAKTERIEN**

METHOD FOR DETERMINING THE AMOUNT OF ANAMMOX BACTERIA

PROCÉDÉ DE DÉTERMINATION DE LA QUANTITÉ DE BACTÉRIES ANAMMOX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.08.2014  AT 505792014**

(43) Veröffentlichungstag der Anmeldung:
**28.06.2017  Patentblatt 2017/26**

(73) Patentinhaber: **Universität Innsbruck 6020 Innsbruck (AT)**

(72) Erfinder:
• **PÜMPEL, Thomas A-6152 Trins (AT)**
• **PODMIRSEG, Sabine Marie A-6020 Innsbruck (AT)**

(74) Vertreter: **Schwarz & Partner Patentanwälte OG Patentanwälte Wipplingerstraße 30 1010 Wien (AT)**

(56) Entgegenhaltungen:
**WO-A1-00/05176**

• SINCLAIR P R ET AL: "Measurement of heme concentration.", CURRENT PROTOCOLS IN TOXICOLOGY / EDITORIAL BOARD, MAHIN D. MAINES (EDITOR-IN-CHIEF) ... [ET AL.] MAY 2001, Bd. Chapter 8, Mai 2001 (2001-05), Seite Unit 8.3, XP002748593, ISSN: 1934-9262 in der Anmeldung erwähnt

• BERRY E A ET AL: "Simultaneous determination of hemes a, b, and c from pyridine hemochrome spectra", ANALYTICAL BIOCHEMISTRY, ACADEMIC PRESS INC, NEW YORK, Bd. 161, Nr. 1, 15. Februar 1987 (1987-02-15), Seiten 1-15, XP024819308, ISSN: 0003-2697, DOI: 10.1016/0003-2697(87)90643-9 [gefunden am 1987-02-15]

• TANG C J ET AL: "Performance of high-loaded ANAMMOX UASB reactors containing granular sludge", WATER RESEARCH, ELSEVIER, AMSTERDAM, NL, Bd. 45, Nr. 1, 1. Januar 2011 (2011-01-01), Seiten 135-144, XP027536687, ISSN: 0043-1354 [gefunden am 2010-08-17]

• TING-TING CHEN ET AL: "Growth and metabolism characteristics of anaerobic ammonium-oxidizing bacteria aggregates", APPLIED MICROBIOLOGY AND BIOTECHNOLOGY, Bd. 97, Nr. 12, 30. August 2012 (2012-08-30), Seiten 5575-5583, XP055223359, DE ISSN: 0175-7598, DOI: 10.1007/s00253-012-4346-z

• SABINE MARIE PODMIRSEG ET AL: "Comparative evaluation of multiple methods to quantify and characterise granular anammox biomass", WATER RESEARCH, ELSEVIER, AMSTERDAM, NL, Bd. 68, 15. Oktober 2014 (2014-10-15), Seiten 194-205, XP029103121, ISSN: 0043-1354, DOI: 10.1016/J.WATRES.2014.10.005

• ALI M ET AL: "The increasing interest of ANAMMOX research in China: Bacteria, process development, and application", BIOMED RESEARCH INTERNATIONAL 2013 HINDAWI PUBLISHING CORPORATION USA, Bd. 2013, 2013, XP002748594, ISSN: 2314-6133 in der Anmeldung erwähnt

**Beschreibung**

Die vorliegende Erfindung betrifft ein Verfahren zur Ermittlung der Menge an Anammox-Bakterien in Bioreaktoren.

STAND DER TECHNIK

Anammox-Bakterien sind in der Lage, durch anaerobe Ammonium-Oxidation Ammonium ($NH_4^+$) und Nitrit ($NO_2^-$) zu molekularem Stickstoff ($N_2$) umzusetzen. Daher werden Anammox Bakterien für die Stickstoff-Entfernung in Kläranlagen technisch eingesetzt. Eine Kläranlage dieser Art beschreibt die WO 2007/033393 A1. Da der Betrieb einer Kläranlage mit Anammox-Bakterien relativ aufwändig ist, wurden bis vor Kurzem lediglich hoch mit Stickstoff belastete Abwässer mit Anammox-Bakterien behandelt. Ziel ist es allerdings, auch bei gering mit Stickstoff belasteten Abwässern in kommunalen Kläranlagen Anammox-Bakterien einzusetzen.

Für den optimalen Betrieb von Kläranlagen mit Anammox-Bakterien (= AnAOB anoxic ammonium oxidizing bacteria) ist eine quantitative Erfassung dieser Mikroorganismen wichtig. Der Grund hierfür liegt darin, dass Anammox-Bakterien eine sehr lange Verdoppelungszeit von 11 bis 20 Tagen aufweisen (Jetten et al. 2009), was verglichen mit anderen Bakterien in Kläranlagen (z.B. AerAOB (= aerobic amonnium oxidizing bacteria) oder NOB (= nitrite oxidizing bacteria)) ein sehr langer Zeitraum ist (Jetten et al. 2001). Während sich bei Bakterien mit Verdoppelungszeiten im Bereich von Stunden störende Einflüsse rasch auswirken, kann man bei diesen auch rasch gegensteuern und innerhalb kürzester Zeit wieder einen physiologischen Gleichgewichtszustand bzw. stationären Zustand erreichen. Bei Anammox-Bakterien macht sich ein störender Einfluss auf das Wachstum erst nach Tagen oder Wochen bemerkbar, sodass ein Gegensteuern meist zu spät kommt. Bis die Zahl der Anammox-Bakterien in der Kläranlage nach dem Gegensteuern wieder Normalniveau erreicht, vergehen weitere Tage oder Wochen, sodass die Kläranlage über einen erheblichen Zeitraum keine optimale Stickstoff-Entfernung leistet.

KURZBESCHREIBUNG DER ERFINDUNG

Aufgabe der vorliegenden Erfindung ist daher, eine Methode bereit zu stellen, um die Anzahl von Anammox-Bakterien leicht bestimmen zu können, damit der Zustand in einem Bioreaktor, insbesondere in einer Kläranlage, beobachtet werden kann.

Gelöst wird diese Aufgabe durch ein Verfahren zur Ermittlung der Menge an Anammox-Bakterien ohne Häm-Derivatisierung mit Pyridin und Oxidierung von Häm und anschließender vergleichender Messung von oxidiertem und reduziertem Häm in Bioreaktoren, insbesondere Klärbecken, umfassend die Schritte

i) Entnehmen einer Probe aus dem Bioreaktor, insbesondere Klärbecken; danach
ii) gegebenenfalls Abtrennen der festen Bestandteile von den flüssigen Bestandteilen, bei Vorhandensein von rotgefärbten Verunreinigungen im Wellenlängenbereich 500 nm bis 600 nm, wobei der Überstand verworfen wird;
iii) Versetzen der Probe mit Metallhydroxid;
iv) gegebenenfalls Suspendieren der festen Bestandteile in der alkalischen Lösung;
v) Erwärmen der Proben auf zumindest 60°C;
vi) Abtrennen fester Bestandteile;
vii) Zugabe eines Salz der dithionigen Säure als Reduktionsmittel zur flüssigen Phase; danach
viii) Messen der Lichtdurchlässigkeit der flüssigen Phase in einem Spektrophotometer bei drei Wellenlängen im Bereich 500 bis 600 nm wobei die Wellenlängenmessung der ersten Wellenlänge bei 530 bis 540 nm, vorzugsweise 535 nm, der zweiten Wellenlänge bei 545 bis 555 nm, vorzugsweise bei 550 nm und der dritten Wellenlänge bei 565 bis 575, vorzugsweise bei 570 nm erfolgt;
ix) Vergleichen der gemessenen Lichtdurchlässigkeit mit einem Referenzspektrum eines Häm-Proteins mit bekanntem Häm-Gehalt.

In Schritt (i) wird zunächst eine repräsentative Probe entnommen.

Wenn die Flüssigkeitsmatrix keine nennenswerte Messstörung erzeugt, was durch Vorversuche ermittelbar ist, wird in Schritt (iii) die Probe mit einem Metallhydroxid versetzt. Dazu können direkt zur Probe soviel hochkonzentrierte Lösung von oder festes Methallhydroxid zugegeben werden, dass die Endkonzentration an $OH^-$ vorzugsweise 1 mol/L beträgt. Bevorzugt werden Metallhydroxide der Alkalimetallionen, insbesondere NaOH eingesetzt.

Wenn die Flüssigkeitsmatrix eine nennenswerte Messstörung erzeugt, wird nach Schritt (i) und vor Schritt (iii) der zusätzliche Schritt (ii) Abtrennen der festen Bestandteile von den flüssigen Bestandteilen, durchgeführt. Dieser Schritt ist allerdings nur notwendig, wenn es nennenswerte Mengen an rotgefärbten Verunreinigungen (Wellenlängenbereich 500 nm bis 600 nm) gibt. Das Abtrennen der festen Bestandteile erfolgt z.B. durch Zentrifugation. Hierfür können Zentrifugenröhrchen eingesetzt werden. Vorteilhaft hat sich eine Zentrifugation bei einer Zentrifugalbeschleunigung von 5.000 g für 3 min erwiesen. Der Überstand wird dann verworfen und die Probe mit einer Alkalie versetzt (Schritt (iii)). Hierfür wird der Probenbehälter bevorzugt auf das Originalvolumen aufgefüllt und der feste Rückstand resuspendiert.

In Schritt (v) erfolgt eine Erwärmung der Proben auf zumindest 60°C, bevorzugt zumindest 70°C, besonders bevorzugt zwischen 90 und 110°C. Die Erwärmung erfolgt vorzugsweise für zumindest eine Minute, bevorzugt für etwa 2 Minuten, besonders bevorzugt für etwa 10 min. Es kann auch länger, beispielsweise 20 Minuten erwärmt werden. Die Erwärmung kann z.B. in einem Wasserbad oder in einer Heizeinrichtung, beispielsweise einem Heizblock, erfolgen. Während oder nach dem Erwärmen ist es vorteilhaft das Probengefäß zu schütteln und abkühlen zu lassen.

In Schritt (vi) erfolgt ein Abtrennen fester Bestandteile. Hierfür kann erneut ein Zentrifugationsschritt vorgesehen sein, beispielsweise bei einer Zentrifugalbeschleunigung von 5.000 g für 3 min.

Die vorgehenden Schritte dienen dazu, das in den Anammox-Bakterien vorhandene Häm in die Lösung freizusetzen. Im darauffolgenden Schritt (vii) erfolgt die Zugabe eines Reduktionsmittels zur flüssigen Phase. Dazu ist eine Menge vorgesehen, die ausreicht, um Häm in die reduzierte Form im Wesentlichen vollständig zu überführen. Praktikabel hat es sich erwiesen, zu 1 ml der flüssigen Phase 100 $\mu$l einer frisch hergestellten Lösung von Reduktionsmittel wie Natriumdithionit (200 g/L in Natronlauge 1 mol/L) zuzugeben und zu vermischen, um das Häm zu reduzieren. Alternativ dazu kann die entsprechende Menge an Reduktionsmittel (z.B. Natriumdithionit) in Pulverform zugegeben werden.

Im nächsten Schritt (viii) erfolgt das Messen der Lichtdurchlässigkeit der flüssigen Phase in einem Spektrophotometer bei drei Wellenlängen im Bereich 500 bis 600 nm. Konkret wurde im vorliegenden Fall die Extinktion der Flüssigkeit im Spektralphotometer bei 535, 550 und 570 nm gemessen. Alternativ dazu kann natürlich auch die Transmission gemessen werden.

Im abschließenden Schritt (ix) erfolgt der Vergleich der gemessenen Lichtdurchlässigkeit mit einem Referenzspektrum. Damit kann auf die konkrete Menge an Häm geschlossen werden, die ein Maß für das Vorhandensein von Anammox-Bakterien im Abwasser ist.

Die Peakhöhe von Häm kann z.B. bei 550 nm gegen die, durch die beiden Messpunkte bei 535 und 570 nm definierte Basislinie, berechnet werden (3-Wellenlängenkalibrationsfunktion). Zur Kalibration kann jedes beliebige Häm-Protein mit bekanntem Häm-Gehalt verwendet werden, z.B. Cytochrom c aus Pferdeherz (Sigma C-2506; M = 12.384 g / mol).

Die Wellenlängenmessung der ersten Wellenlänge erfolgt bei 530 bis 540 nm, vorzugsweise 535 nm, der zweiten Wellenlänge bei 545 bis 555 nm, vorzugsweise bei 550 nm und der dritten Wellenlänge bei 565 bis 575, vorzugsweise bei 570 nm.

Weiters kann vorgesehen sein, dass das Abtrennen der festen Bestandteile mittels Zentrifugation erfolgt. Zusätzlich oder alternativ dazu kann ein Filtrationsschritt vorgesehen sein.

Die vorliegende stellt eine effiziente und einfache Methode zur indirekten Quantifizierung von Anammox-Bakterien zur Verfügung. Die Methode bedient sich dabei der Tatsache, dass Anammox-Bakterien eine große Menge an Häm-Proteinen aufweisen. Der Häm-Gehalt wiederum korreliert mit der Aktivität der Anammox-Bakterien und kann somit als indirekte Messgröße für die Quantifizierung herangezogen werden.

Im Unterschied zu bereits bekannten Verfahren zur Quantifizierung von Häm im medizinischen Bereich (Sinclair et al., Curr Protoc Toxicol. 2001 May; Chapter 8:Unit 8.3), wurde das vorliegende Verfahren für Anammox-Bakterien optimiert. Die Erfindung optimiert das Verfahren von Sinclair et al., indem zwei Schritte weggelassen werden. Zum ersten wird die Häm-Derivatisierung mit Pyridin weggelassen, zum zweiten wird auf eine Oxidierung des Häm und anschließender vergleichender Messung von oxidiertem und reduziertem Häm verzichtet.

Es wurde im vorliegenden Fall die Häm-Extraktion aus den Anammox-Bakterien bezüglich Laugenstärke, Temperatur und Einwirkdauer optimiert. Bei dem von Sinclair et al. vorgeschlagenen Verfahren erfolgt eine Häm-Derivatisierung mit giftigem Pyridin. Eine solche ist beim vorliegenden Verfahren nicht erforderlich. Anstatt der ebenfalls von Sinclair et al. verwendeten Bestimmung der optischen Differenzspektren von oxidiertem und reduziertem Häm wird die einfachere Messung nur des reduzierten Häms bei - vorzugsweise drei - Wellenlängen vorgeschlagen.

Die Reihenfolge der Schritte iii) (bzw. gegebenenfalls ii)) bis vii) kann auch variiert werden, während Schritt i) vor den Schritten ii) bis vii) durchgeführt werden muss und die Schritte viii) bis ix) nach den Schritten ii) bis vii) durchgeführt werden muss.

D.h. das Versetzen der der Probe mit Alkalie kann auch nach der Zugabe des Reduktionsmittels zur flüssigen Phase erfolgen; auch die Erwärmung kann z.B. nach Zugabe von Reduktionsmittel und Alkalie erfolgen. Bevorzugt erfolgt das Abtrennen fester Bestandteile allerdings nach der Zugabe von Alkalie und/oder Reduktionsmittel.

Daher ist das erfindungsgemäße Verfahren in jedem Standardlabor mit einem üblichen Spektralphotometer durchführbar. Damit die Messung auch in einer Kläranlage mit nur einfachen Laborgeräten ausgeführt werden kann, wird erfindungsgemäß weiters ein Kit, umfassend ein Probengefäß, eine alkalische Lösung, ein Reduktionsmittel und ein Spektrophotometers, welches zumindest bei drei Wellenlängen im Bereich 500 bis 600 nm betreibbar ist, vorgeschlagen.

Dieses Kit kann zusätzlich durch eine Heizeinrichtung gekennzeichnet sein.

Weiters kann das Kit eine Abtrennvorrichtung für feste Stoffe aufweisen. Beispielsweise kann dies ein Filter oder eine Zentrifuge sein.

Die erforderlichen Chemikalien für den Kit können auch vorgelegt sein. Damit verringert man die erforderlichen Hantierschritte bei der Benutzung. Daher ist in einer Ausführungsvariante vorgesehen, dass das Kit ein - vorzugsweise verschlossenes - Probengefäß umfasst, in welchem eine Alkalie oder alkalische Lösung vorgelegt ist.

Weiters kann vorgesehen sein, dass das Kit ein - vorzugsweise verschlossenes - Probengefäß umfasst, in welchem das Reduktionsmittel vorgelegt ist.

Vorzugsweise ist das Probengefäß, in welchen das Reduktionsmittel vorgelegt ist, eine Küvette für das Spektrophotometer.

Die Bestimmung von Aktivität bzw. Biomasse von Anammox-Bakterien in Kläranlagen-Labors ist momentan nur begrenzt möglich. Bei der Applikation im Kläranlagen-Hauptstrom sind die gängigen Methoden, wie Bestimmung der Umsatzraten oder die gravimetrische Analyse, ungeeignet, da alternative Stoffumsätze zumindest gleichwertig ablaufen und so die Anammox-Aktivität überlagern. Die Analyse spezifischer Inhaltsstoffe zur Abschätzung von Aktivität bzw. Biomasse der Anammox-Bakterien, wie Häm oder DNA, war bisher entsprechend ausgestatteten Forschungslabors vorbehalten und daher nicht für das tägliche Monitoring vor Ort geeignet. Die vorliegende Erfindung bietet den Vorteil, dass eine Quantifizierung über eine Häm-Bestimmung ohne großen Aufwand in jedem Standard-Kläranlagenlabor durchgeführt werden kann. Die Erfindung ermöglicht damit das Monitoring von Anammox-Bakterien im Routine-Labor vor Ort, benötigt nur Standardchemikalien bzw. Standardausrüstung, und ist daher wesentlich ökonomischer als die Analyse durch Speziallabors. Durch ein kontinuierliches Monitoring können frühzeitig Betriebsstörungen erkannt werden und Gegenmaßnahmen eingeleitet werden.

DETAILBESCHREIBUNG UND BEISPIELE

Zur Illustration wird auf die Figuren verwiesen:

Fig. 1 zeigt die nicht-metrische multidimensionale Skalierung der AC/AnAOB Biomassenquantifizierung für sechs Probentypen, SL, SL-OF, SL-UF, B, B-OF und B-UF. Alle sechs quantitativen Methoden, GA, PT, HQ, AM, qPCR und CC wurden für die Berechnung inkludiert. Proben aus der gleichen Gruppe (Parallelen) sind über Linien verbunden, die die konvexe Hüllenfläche der NM-MDS Resultate widerspiegeln.

Fig. 2 zeigt den Einfluss der NaOH-Konzentration auf die Häm-Ausbeute aus Anammox-Biomasse (4 min Aufschlusszeit).

Fig. 3 zeigt den Zeitverlauf der Hämextraktion aus Anammox-Biomasse in 1 M NaOH bei 100°C.

Fig. 4 zeigt die Optimierung von Art, Alter und Konzentration des Reduktionsmittels (Na-DT = Natriumdithionat).

Fig. 5 zeigt die Zugabe von Natriumdithionit als Pulver bzw. frisch gelöst in 1 M NaOH bei jeweils gleicher Endkonzentration.

Fig. 6 zeigt die Stabilität des Hämochroms nach Reduktion mit NaDT.

Fig. 7 zeigt eine Beispielmessung am DEMON®-Reaktor der Kläranlage Tobl/Bruneck und demonstriert die Anreicherung der Anammox-Biomasse im Zyklon-Unterlauf.

**Vergleich von verschiedenen Methoden zur Bestimmung von Masse oder Aktivität von Anammox-Bakterien**

Aufgrund zum Teil sehr außergewöhnlicher physiologischer und morphologischer Eigenschaften kommen die folgenden, sehr unterschiedlichen Untersuchungsmethoden in Frage:

- Quantifizierung funktioneller oder phylogenetischer Gene mit molekularbiologischen Techniken
- Chemisch-analytische Quantifizierung charakteristischer Zellinhaltsstoffe wie Membranbestandteile, vor allem Ladderane
- Messung der charakteristischen physiologischen Aktivität, insbesondere des Umsatzes von Ammonium mit Nitrit zu molekularem Stickstoff und Nitrat
- Quantifizierung und Vermessung der typischen granulären Strukturen der Anammoxbakterien.

Trotz der Vielfalt an analytischen Möglichkeiten werden heute fast nur die molekularbiologischen Techniken und die Messung der physiologischen Aktivität angewandt. Insbesondere für die Routineüberwachung in der Kläranlage fehlen jedoch aussagekräftige, zuverlässige und robuste aber dennoch einfach vor Ort mit Standardausrüstung durchzuführende Methoden für die Kontrolle der Anammox-Population.

Proben der Kläranlage Strass im Zillertal (AT) wurden mit sechs verschiedenen Methoden untersucht, die Ergebnisse statistisch ausgewertet, und die Eigenschaften der Methoden, insbesondere deren Vor- und Nachteile, gegenübergestellt:

1. Gravimetrische Analyse von Anammox-Granulae (GA)
2. Quantifizierung des Häm-Gehaltes (HQ)
3. Zählung und Vermessung der Granulae mittels Coulter Counter (CC)
4. Zählung und Vermessung der Granulae mittels Bildanalyse (PT)
5. DNA Extraktion und quantitative Polymerase-Kettenreaktion (qPCR)
6. Physiologische Aktivitätsmessung (AM)

**Material & Methoden**

**Proben, Probenahme und -Vorbehandlung**

Im Rahmen dieser Erfindung wurden Proben der Kläranlage Strass im Zillertal untersucht. Die Proben stammen aus verschiedenen Teilanlagen und unterscheiden sich daher deutlich bezüglich ihrer Probenmatrix:

a) DEMON® - Reaktor, behandelt hoch mit Stickstoff belastetes Faulschlammpresswasser, enthält große Mengen an Anammox-Bakterien
b) Belebungsbecken im Hauptstrom, das seit 2011 mit Anammox-Biomasse aus dem DEMON® - Reaktor dotiert wird. Hier wird relativ gering mit Stickstoff belastetes, kommunales Abwasser behandelt, die Anammox-Bakterien sind gegenüber anderen Bakterienpopulationen wesentlich geringer konzentriert.

Außerdem wurden an jeder Anlage drei verschiedene Fraktionen an dem jeweils installierten Hydrozyklon entnommen: i) Zyklon-Zulauf (= Reaktorinhalt), ii) Zyklon-Unterlauf (= angereicherte Anammox-Fraktion, die in den Reaktor zurückgeführt wird), iii) Zyklon-Oberlauf (= abgereicherte Anammox-Fraktion, abgezogener Überschuss-Schlamm).

Tabelle 1: Probenbezeichnungen

| Anlage | Fraktion | Probenbezeichnung |
|---|---|---|
| DEMON® | Becken | SL |
| | Zyklon-Oberlauf | SL-OF |
| | Zyklon-Unterlauf | SL-UF |
| Hauptstrom | Belebungsbecken | B |
| | Zyklon-Oberlauf | B-OF |
| | Zyklon-Unterlauf | B-UF |

Von jeder Probe wurden 3 Parallelen zu je 1L repräsentativ entnommen. Die weitere Probenteilung für die verschiedenen Analysen erfolgte in einem voll durchmischten Rührgefäß, um vor allem die rasche Sedimentation der granulären Biomasse zu unterbinden (Gefäß mit Schikanen, Rushton-Rührer). Je nach Methode wurden die Teilproben bis zur Untersuchung tiefgefroren (qPCR), bei 4°C gelagert (GA, CC, PT) oder sofort am gleichen Tag untersucht (AM, HQ).

**Gravimetrische Analyse (GA)**

Die Masse abfiltrierbarer Stoffe wurde durch Filtration durch Glasfaserfilter (Macherey&Nagel GF5) und Trocknung über Nacht bei 105°C bestimmt. Der Glühverlust als Maß für den organischen Anteil wurde nach 3 h bei 550°C analysiert.
Bei den Proben aus dem DEMON®-Reaktor wurden die Anammox-Granulae durch ein Analysensieb mit 0,125 mm Maschenweite nass abgesiebt, getrocknet und gewogen. Bei den Hauptstromproben war dies wegen der schleimigen Biomasse nicht möglich.

**Quantifizierung des Häm-Gehaltes (HQ)**

Je 1,5 mL der Proben wurden in 2 mL-Mikroreaktionsgefäßen 3 min bei 5000 g zentrifugiert. Der Überstand wurde verworfen und durch 1 M NaOH ersetzt. Das Pellet wurde resuspendiert, die Gefäße für 2 min in kochendes Wasser gestellt und im Anschluss für 10 min unter gelegentlichem Aufschütteln abkühlen lassen. Restpartikel wurden bei 5000 g für 3 min abzentrifugiert, und 1 mL vom klaren Überstand wurde in Halbmikro-Photometerküvetten überführt. Zur Reduktion des extrahierten Häms wurden 100 $\mu$L einer frisch bereiteten Lösung von Natriumdithionit (200 g/L in 1 M NaOH) eingemischt.

Nach 5 min wurden die Extinktionen bei 535, 550 und 570 nm mit einem Spektrophotometer gemessen. Der Peak bei 550 nm wurde auf die Basislinie, die durch die Extinktionen bei 535 und 570 nm definiert ist, bezogen.

Die Analyse wurde mit Cytochrom c aus Pferdeherz (enthält 1 Häm pro Molekül; Sigma C-2506) kalibriert.

Berechnung der Peakhöhe bei 550 nm:

$$Peakh\ddot{o}he\ (AU) = E_{550} - E_{535} - \frac{E_{570} - E_{535}}{570nm - 535nm} * (550nm - 535nm)$$

$E_{535}$, $E_{550}$, $E_{570}$...gemessene Extinktionen (AU) bei der jeweiligen Wellenlänge

## Zählung und Vermessung der Granulae mittels Bildanalyse "Particle Tracking" (PT)

Je 15 mL Probe wurden in Petrischalen gefüllt und diese mit einem Flachbettscanner bei 600 dpi Auflösung gegen einen homogenen weißen Hintergrund aufgenommen. Die Proben wurden so stark verdünnt, dass im Bild keine Überlagerungen von Partikeln auftraten.

Die Bilder wurden mit einem Bildanalyseprogramm (Fiji) so bearbeitet, dass die Anammoxgranulae aufgrund ihres Farbunterschieds zum Hintergrund und zu anderen Partikeln getrennt erfasst werden konnten. Die erkannten Granulae wurden gezählt und von jedem Granulum wurde die Querschnittsfläche berechnet. Anzahl der Granulae und die Summe der Querschnittsflächen wurden auf das eingesetzte Probenvolumen bezogen und als Maß für die Anammox-Biomasse genutzt.

## Zählung und Vermessung der Granulae mittels Coulter Counter (CC)

Als Alternative zur Bildauswertung wurde auch ein Partikelanalysator nach dem Coulter-Prinzip verwendet (Coulter 1956). Dabei werden die Partikel durch eine Messöffnung gesaugt und die dabei auftretende Erhöhung des elektrischen Widerstands der Öffnung durch Querschnittsverengung wird gemessen und ausgewertet. Im Gegensatz zur Bildanalyse erfasst der Coulter Counter nur jenes Volumen eines Teilchens, das elektrisch vom umgebenden Elektrolyten isoliert ist. Bakterielle Schleime und Kavitäten der Anammox-Granulae werden so nicht erfasst, sondern die Summe intakter Zellen.

Die Proben wurden in Proben-isotoner Salzlösung so weit verdünnt, dass immer nur einzelne Partikel durch die Öffnung gesaugt werden. Die Messungen wurden mit einem Multisizer II (Coulter Beckman Inc.) mit einem Messrohr mit 1000 $\mu$m Messöffnung durchgeführt.

Die Größenkalibration erfolgte mit kugelförmigen Styroldivinylbenzol-Partikeln mit 320 $\mu$m Modal-Durchmesser.

## DNA Extraktion und quantitative Polymerase-Kettenreaktion (qPCR)

Die quantitative Polymerase-Kettenreaktion (qPCR) wurde zur Bestimmung der 16S rRNA Genkopien-Anzahl verwendet. Zuvor wurde die DNA mit dem DNeasy® Blood & Tissue Kit, entsprechend dem "Pretreatment for Gram-Positive Bacteria" Protokoll extrahiert (QIAGEN; Handbuch 07/2006). Um die DNA-Gesamtausbeute zu erhöhen, wurden zu Beginn noch drei Gefrier-Tau-Zyklen (Flüssigstickstoff / 65°C Wasserbad; jeweils 5 Minuten) durchgeführt. Zusätzlich wurde nach der chemischen Lyse laut Herstellerangaben (20 mg/mL Lysozym und Proteinase K) ein mechanischer Aufschluss inkludiert, bei dem die Probe in PowerBead Gefäße (MO BIO Laboratories, Inc.) überführt und bei 60 Hz für 4 Minuten in einem Horizontalschüttler geschüttelt wurde. Anschließend wurde die Probe bei 8000 g 3 Minuten zentrifugiert und das Protokoll fortgesetzt. Die finale Elution fand in 100 $\mu$L AE Puffer (auf 60°C erwärmt) in zwei aufeinanderfolgenden Schritten (2 x 50 $\mu$L) statt.

## Physiologische Aktivitätsmessung (AM)

Die Stoffwechselaktivität der Anammox-Bakterien wurde quantifiziert als Umsatzrate der Summe der drei wesentlichen gelösten Stickstoff-Spezies Ammonium, Nitrit und Nitrat, wobei unter ungestörten Bedingungen folgende Reaktionsgleichung gilt:

$$NH_4^+ + 1,32\ NO_2^-\ 0,132\ HCO_3^- + 0,512\ H^+ \Rightarrow 1,02\ N_2 + 0,26\ NO_3 + 0,132\ CH_2O_{0,5}N_{0,15} + 2,19\ H_2O$$

Die zu untersuchenden Proben wurden auf ausreichende Startkonzentrationen der Substrate ($NH_4$: 100 mg N/L mit $NH_4HCO_3$, $NO_2$: 70 mg N/L mit $NaNO_2$), und der pH-Wert auf 7,2 eingestellt. Jeweils 100 mL wurden in gasdicht verschließbare 100 mL-Erlenmeyerkolben gefüllt und der gelöste Sauerstoff durch Einblasen von Stickstoffgas ausgetrieben. Die Gefäße wurden verschlossen und unter Schütteln bei 30°C inkubiert. Über insgesamt 4 h wurden in 30 min Intervallen 1 mL-Proben entnommen, zentrifugiert, und im Überstand die Konzentrationen von Nitrit, Nitrat und Ammonium gemessen (jeweils mit Nanocolor Kits von Macherey&Nagel). Die Konzentrationen wurden pro Messpunkt addiert (in mg N/L) und aus dem linearen Bereich des zeitlichen Konzentrationsverlaufs die Steigung berechnet, die als Maß für die Aktivität herangezogen wurde (volumetrische N-Umsatzrate in mg N/(L.h)). Zusätzlich wurde die spezifische N-Umsatzrate durch Bezug auf die in der Probe enthaltene Biomasse-Trockensubstanz berechnet (mg N/(L.h.g TS)).

### Statistische Analysen

Alle statistischen Analysen wurden mit der Software PAST (Version 2.17) (Hammer et al. 2001) durchgeführt. Die Auftrennung der verschiedenen Probentypen unter Verwendung aller untersuchten Methoden wurde anhand einer nicht-metrischen multidimensionalen Skalierung veranschaulicht. Hierfür wurden die Daten log-transformiert und der Bray-Curtis Index verwendet. Die Korrelation zwischen den verschiedenen Methoden wurde durch eine MANTEL Permutationsanalyse, basierend auf Bray-Curtis Distanzmatrizzen, für jede Methode und alle Probentypen veranschaulicht. Zur Überprüfung der Reproduzierbarkeit der Häm-Methode wurden der Varianzkoeffizient (CV(%)) bestimmt und für jede Probenmatrix die Mittelwerte berechnet und miteinander verglichen.

### Ergebnisse

### Allgemeiner Vergleich der sechs verwendeten Methoden

Tabelle 2 fasst alle Analyseergebnisse und zusätzliche Daten zu den einzelnen Probentypen (Trockensubstanz (TS), organische Trockensubstanz (oTS), Granulenanzahl) zusammen. Die stark rot gefärbten AC-Granulen (Anammox Consortium: AnAOB, AerAOB und andere mögliche mikrobielle Gruppen, die ebenso in den Granulen aggregiert sein können, wie z.B. NOB und *Chloroflexi*) aus der Kläranlage Strass weisen eine relativ heterogene Struktur auf. Kryotomschnitte zeigen, dass das Innere dieser Granulen zu einem großen Teil aus Hohlräumen besteht (Daten nicht ausgewiesen).

Aufgrund der Probenauswahl und der Dichtefraktionierung mittels Hydozyklonen der Anlagen kann von folgender grundlegenden Reihung der Proben bezüglich Anammox-Biomasse ausgegangen werden: SL-UF > SL > SL-OF > B-UF > B > B-OF (Anammox im DEMON®-Reaktor (SL) viel aktiver als im Hauptstrom (B), und Anammox-Anreicherung jeweils im Zyklon-Unterlauf). Diese Hypothese galt es mit den verwendeten Methoden zu bestätigen.

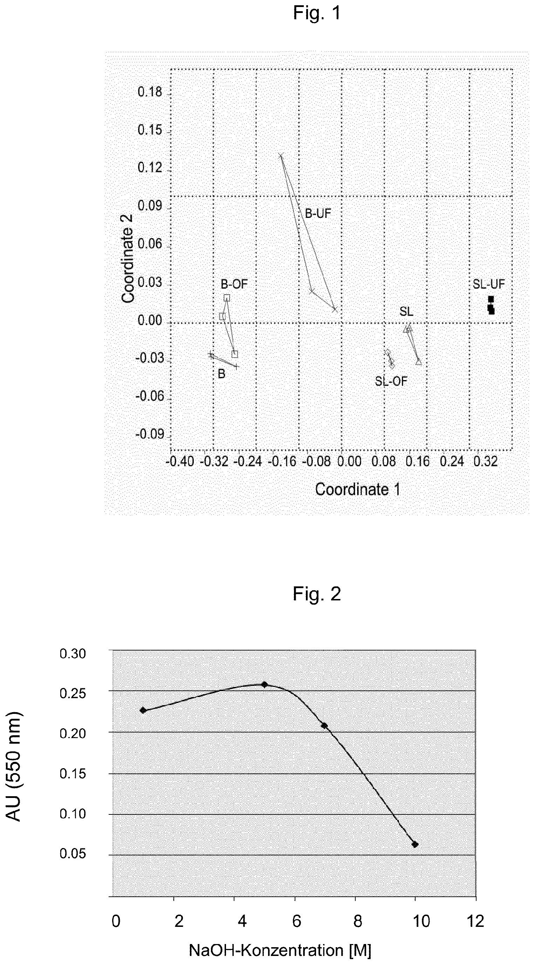

Alle sechs Methoden waren in der Lage, die verschiedenen Probenmatrizzen und Hydrozyklon-Fraktionen voneinander zu unterscheiden (nicht-metrische multidimensionale Skalierung (NM-MDS), Fig. 1). Ein niederer Stress-Wert von < 0,02 deutete auf eine realistische Abbildung der Originaldaten in der NM-MDS hin. Die $R^2$-Werte waren 0,99 (Koordinate 1) beziehungsweise 0,00007 (Koordinate 2). Es zeigte sich, dass die SL-Proben besser voneinander zu unterscheiden waren, als z.B. die Proben B und B-OF, bei denen es entlang der X-Achse ($R^2 = 0,99$) zu einer Überlappung kam. Proben, die generell mehr Anammox-Biomasse enthielten (SL-Matrix), konnten insgesamt besser aufgetrennt werden.

Betrachtet man die Ergebnisse aller sechs Methoden einzeln, so konnten alle die SL-Proben richtig einteilen, für die B-Matrix war dies nur mittels qPCR und HQ möglich (Tabelle 3). Die Quantifizierung mittels CC wurde nur für die SL-Matrix durchgeführt, da sich die Abtrennung der Granulen aus dem Belebtschlamm bei B-Proben als sehr komplex herausstellte. Zur Überprüfung der Reproduzierbarkeit bzw. Varianz der Häm-Methode wurde der Varianzkoeffizient bestimmt. Hierbei zeigte sich, dass der durchschnittliche CV für beide Probenmatrizzen etwa gleich war, nämlich 4,45 (SL), beziehungsweise 5,20 (B), und diese Methode somit in deutlich unterschiedlichen Probenmattrizzen zu ähnlich reproduzierbaren Daten führte.

Betrachtet man alle Methoden hinsichtlich ihrer Eignung, nur AnAOB Biomasse zu quantifizieren, so ist der qPCR Ansatz, der die 16S rRNA der *Planctomycetales* anvisiert, die genaueste Methode. Möchte man allerdings die AC Biomasse bestimmen, so sind HQ, PT und CC ebenso geeignet. Die PT-Methode birgt gegenüber dem CC-Ansatz den Vorteil der zusätzlichen Farbselektion und dadurch besseren Identifikation von AC-Granulen, wodurch diese Methode auch für komplexere Matrizzen, z.B. Belebtschlamm mit eingebetteten Granulen, geeignet ist. Beide Methoden liefern neben der Quantifizierung der AC-Biomasse eine zusätzliche qualitative Auswertung über die Größenverteilung der Granulen. Die GA wurde aufgrund von drei falschen Einteilungen der Proben als ungenaueste Methode klassifiziert (siehe Tabelle 3).

Tabelle 4 fasst die wichtigsten Vor- und Nachteile jeder Methode zusammen und liefert weitere Informationen über die durchschnittliche Dauer der Messungen, Detektionslimits und den Preis pro Analyse.

In Tabelle 5, 6 und 7 sind die Ergebnisse der Korrelationsanalysen zwischen allen Methoden gezeigt, die wie folgt zusammengefasst werden können: die höchste Korrelation wurde für die SL-Matrix erreicht, mit signifikanten Pearson's Korrelationskoeffizienten für alle Methoden > 0,71. Exkludiert man die qPCR-Methode, so erzielt man sogar Werte > 0,96. Betrachtet man beide Probenmatrizzen gemeinsam, so erhält man immer noch signifikant positive Korrelationen, wenn auch mit etwas niederen Pearson's Koeffizienten (0,30 - 0,96). Die höchste Korrelation wurde zwischen GA und PT erreicht, die niedersten Werte mit AM. Die Analyse der B-Matrix lieferte für GA, PT und HQ signifikant hochpositive Korrelationen (alle > 0,94; CC nicht untersucht) und deutlich reduzierte, aber positive Korrelationen für qPCR (0,41 - 0,47). Für die AM wurde keine positive Korrelation in der B-Matrix erreicht, eine Tatsache, die im Kapitel "Physiologische Aktivität" genauer beleuchtet wird.

Zusammenfassend soll die Korrelationsanalyse als Orientierungshilfe dienen, um zu sehen, welche Methoden die untersuchten Proben auf ähnliche Art und Weise gruppieren. Dies impliziert jedoch nicht die Richtigkeit der Ergebnisse. Gleichwohl waren HQ und qPCR die einzigen Methoden, die das Probenset wie erwartet einteilen konnten (siehe Tabelle 3), und beide Methoden erzielten einen Korrelationskoeffizienten von 0,81. Eine hohe Korrelation einer anderen Methode mit diesen beiden Methoden lässt deshalb auch auf höhere Exaktheit der Ergebnisse schließen.

Im Folgenden werden zusätzliche Beobachtungen mit Proben aus anderen Kläranlagen angeführt. Diese Ergebnisse deuten auf eine hohe Anwendbarkeit der verwendeten Methoden für andere Probenmatrizzen hin: i) Proben aus einem DEMON®-Becken einer anderen Kläranlage enthielten rote und schwarze Anammox-Granulen; diese konnten aufgrund ihrer unterschiedlichen Dichte in zwei Fraktionen aufgeteilt werden und wurden mittels AM und HQ analysiert. Beide Methoden zeigten hohe Aktivität und Häm-Konzentration in der roten Fraktion und drastisch reduzierte Werte in der schwarzen Fraktion. Dies lässt darauf schließen, dass HQ und PT, aufgrund der Farbselektion, nicht nur zur Quantifizierung sondern auch zur Aktivitätsmessung herangezogen werden können, ii) in einer weiteren Kläranlage, die ebenso Nebenstrom Deammonifikation mittels DEMON®-Technologie betreibt und zusätzlich zwei komplett getrennte biologische Schlammlinien führt, wurde in einer dieser Linien Anammox-Biomasse angereichert und die zweite Linie als Kontrolle unbehandelt gelassen. In der Kontrolllinie konnten nur wenige Anammox-Granulen durch PT nachgewiesen werden (weniger als 0.1%-1% verglichen mit den Ergebnissen der B-Proben (diese Studie), wohingegen Anammox-Granulen in der bioaugmentierten Linie klar detektiert wurden. iii) Häm-Proteine sind allgegenwärtig in der Natur (Gumiero et al. 2011), dennoch waren die HQ-Messwerte in Proben aus Kläranlagen, die keinen zusätzlichen Deammonifikationsprozess betreiben, unter dem Detektionslimit. Dies deutet auf ein reduziertes Risiko falsch positiver Ergebnisse mit dieser Methode hin.

Tabelle 3: Zusammenfassung der Probeneinteilung durch die sechs verwendeten Methoden, verglichen mit der erwarteten Probenreihung; Unterschiede dieser Reihung sind grau markiert;

| Probenbezeichnung | erwartete AC/AnAOB Abundanz* | GA | PT | HQ | AM | qPCR | CC |
|---|---|---|---|---|---|---|---|
| SL | 2 | 2 | 2 | 2 | 2 | 2 | 2 |
| SL-OF | 3 | 3 | 3 | 3 | 3 | 3 | 3 |
| SL-UF | 1 | 1 | 1 | 1 | 1 | 1 | 1 |
| B | 5 | 5 | 6 | 5 | 6 | 5 | n.a. |
| B-OF | 6 | 5 | 5 | 6 | 5 | 6 | n.a. |
| B-UF | 4 | 3 | 4 | 4 | 4 | 4 | n.a. |
| AnAOB Spezifität | Genauigkeit für reine AnAOB Messung | 6 | 3 | 2 | 5 | 1 | 4 |

*Zahlen reflektieren die gereihte, erwartete AC/AnAOB Abundanz, wobei 1 die höchste und 6 die geringste Abundanz darstellt; Zahlen in der untersten Zeile sind von 1 (am besten geeignete Methode) bis 6 (am schlechtesten geeignete Methode) gereiht. n.a....nicht analysiert;

Tabelle 2: Probencharakterisierung mittels TSS, VSS, granulärer-SS (Partikel > 0.125 mm Durchmesser), $PT_{tot}$ Querschnittsfläche (gesamte Probe), $PT_{sieved}$ Querschnittsfläche (Partikel > 0.125 mm und < 1 mm Durchmesser), $PT_{tot}$ (Partikelanzahl Gesamtprobe), $PT_{sieved}$ (Partikelanzahl > 0.125 mm und < 1mm Durchmesser), CC (AC Granulenvolumer gesiebter Proben > 0.125 mm und < 1mm Durchmesser), HQ, qPCR, AM (Gesamt-Stickstoff-Umsatz), $NH_4$-N-, $NO_2$-N-, und $NO_3$-N-Bildungsrate; Mittelwerte (fett gedruckt) und Standardabweichung; n=3 (für CC n=9 (3 technische Parallelen)), n.a.= nicht analysiert;

| | $SS\ [g\ L^{-1}]$ | $VSS\ [g\ L^{-1}]$ | $GA$ granuläre r-SS $[g\ L^{-1}]^*$ | $PT_{tot}$ Querschnitts-fläche $[mm^2\ L^{-1}]$ | $PT_{sieved}$ Quer-schnittsfläche $[mm^2\ L^{-1}]$ gesiebt | $PT_{tot}$ [Par-tikel $L^{-1}$] | $PT$ sieved [Partikel $L^{-1}$] | $CC$ $[mL\ L^{-1}]$ | $HQ$ $[mAU\ L^{-1}]$ | qPCR [Genkopien $L^{-1}$] | $AM$ [mg N $L^{-1}*h^{-1}$] | $NH_4$[mg $NH_4$-N $L^{-1}\ h^{-1}$] | $NO_2$[mg $NO_2$-N $L^{-1}\ h^{-1}$] | $NO_3$[mg $NO_3$-N $L^{-1}\ h^{-1}$] |
|---|---|---|---|---|---|---|---|---|---|---|---|---|---|---|
| **SL** | **2.95** | **2.32** | **0.79** | **283** | **280** | **326267** | **297600** | **40** | **164** | **9.32E+11** | **-36.1** | **-17.2** | **-22.7** | **3.72** |
| | 0.09 | 0.07 | 0.12 | 35.6 | 33.2 | 36116 | 32822 | 0.94 | 12.2 | 7.50E+11 | 4.51 | 4.27 | 0.77 | 0.24 |
| **SL-OF** | **2.19** | **1.77** | **0.44** | **131** | **129** | **184400** | **160933** | **35.8** | **86.4** | **7.01E+11** | **-23.1** | **-10.8** | **-14.6** | **2.22** |
| | 0.06 | 0.05 | 0.00 | 11.3 | 11.2 | 9165 | 8259 | 1.61 | 7.03 | 2.48E+11 | 2.20 | 1.39 | 1.15 | 0.24 |
| **SL-UF** | **8.95** | **6.59** | **4.69** | **4985** | **4965** | **4249333** | **3958667** | **113** | **1143** | **3.72E+12** | **-115** | **-71.3** | **54.7** | **10.9** |
| | 0.51 | 0.32 | 0.19 | 984 | 997 | 691449 | 490067 | 3.00 | 8.68 | 1.19E+12 | 2.33 | 2.95 | 1.93 | 0.52 |
| **B** | **4.25** | **3.05** | **0.07** | **3.62** | **3.46** | **8533** | **6000** | ***n.a.*** | **12.3** | **4.00E+09** | **-0.45** | **3.65** | **-4.11** | **0.01** |
| | 0.61 | 0.44 | 0.01 | 0.55 | 0.66 | 462 | 2227 | | 0.33 | 1.27E+08 | 2.83 | 2.53 | 0.31 | 0.01 |
| **B-OF** | **4.20** | **3.03** | **0.07** | **5.97** | **4.21** | **47333** | **13600** | ***n.a.*** | **11.6** | **2.70E+09** | **-1.20** | **2.57** | **-3.78** | **0.01** |
| | 0.09 | 0.07 | 0.01 | 0.94 | 0.82 | 3585 | 1600 | | 0.51 | 4.03E+08 | 2.43 | 2.64 | 0.55 | 0.01 |
| **B-UF** | **6.71** | **4.53** | **0.79** | **52.3** | **50.1** | **113200** | **76000** | ***n.a.*** | **25.1** | **2.88E+10** | **-3.23** | **4.00** | **-7.22** | **-0.01** |
| | 0.11 | 0.06 | 0.10 | 16.6 | 16.4 | 22087 | 18543 | | 2.14 | 4.45E+10 | 2.77 | 1.18 | 1.70 | 0.02 |

Tabelle 4: Zusammenfassung der verwendeten Methoden und deren Eignung zur AC/AnAOB Quantifizierung

| Methode | methodischer Hintergrund | Vorteile | Nachteile | Detektionslimit | Eignung für granuläre und nicht-granuläre Biomasse | Zeitbedarf pro Probe* | zusätzlicher Laborgeräte-Bedarf | Preis pro Probe (Materialkosten) |
|---|---|---|---|---|---|---|---|---|
| GA | Massenbestimmung granulärer Biomasse >0.125 mm Durchmesser; | schnell, preiswert, geringer Gerätebedarf; | nicht auf die kleinste Fraktion anwendbar (<0.125 mm); ungenau; Siebverstopfung durch (schleimigen) Hauptstromschlamm | nicht definiert | nur granuläre Biomasse | 5-10 min | Analysesiebe, Trocknungswaage oder Trockenschrank | < 0.50 € |
| PT | Partikelanzahl/Querschnittsflächenbestimmung durch Bildanalyse; | genaue granuläre AC Abundanz und Biomassenfläche/- volumen**; Granulengrößenverteilung; geringer Gerätebedarf | rote*** Granulenfarbe notwendig für Belebtschlammmatrix; | mit konventionellem Flachbettscanner ca. 50 $\mu$m Partikeldurchmesser; | nur granuläre Biomasse | 10 min | Flachbettscanner, Bildanalyse Software | < 0.50 € |
| HQ | Extraktion und kolorimetrische Bestimmung des Hämochroms | relativ schnell; exakt; preiswert; misst v.a. AnAOB; geringer Gerätebedarf; | keine bekannt | das Protokoll wurde so adaptiert, dass Messungen in beiden Probenmatrizzen möglich waren | beliebig | 5-10 min | Standardzentrifuge, Wasserbad oder Heizblock, Photometer | 1- 2 € |
| AM | Summe der $NO_2^-$, $NO_3^-$, $NH_4$-N Zehrungs-/Produktionsraten; | misst die Aktivität, nicht nur Anwesenheit; | Verzerrung durch Denitrifikantenanwesenheit und Ammonifikation (B); | nicht definiert; bei geringen Raten kann Inkubationszeit verlängert werden; | beliebig | 6h | spezielle Gerät für kommerzielle Kits (Photometer) oder alternative Analysegeräte | ca. 50 € (mit kommerziellen Kits) |
| qPCR | 16S rRNA Gen-Quanitifizierung; | AnAOB spezifisch; wenn RNAbasierend auch als Akitivtätsparameter geeignet; | Extraktionsbias; spezielle Gerätschaft notwendig; | 1.2 $10^4$ Genkopien pro L; | beliebig, aber geeignetes Extraktionsprotokoll wichtig | 8-10 h / 3 h**** | molekularbiolog. Laborausstattung, PCR-cycler, qPCR-cycler | ca. 10-15 € |

| Methode | methodischer Hintergrund | Vorteile | Nachteile | Detektionslimit | Eignung für granuläre und nicht-granuläre Biomasse | Zeitbedarf pro Probe* | zusätzlicher Laborgeräte-Bedarf | Preis pro Probe (Materialkosten) |
|---|---|---|---|---|---|---|---|---|
| CC | Bestimmung der/des Partikelanzahl-/-volumen; Granulen werden Größenkanälen zugeordnet; | exakte AC-Granulen-Abundanz und -volumen**; Granulengrößenverteilung; | spezielle Gerätschaft notwendig; Probenmatrix-abhängig; | ideale Granulengröße 2%-60% der Messöffnung (hier 1000 $\mu$m Durchmesser); | nur granulär | 30-45 min | Coulter Counter | < 1 € |

* ca. Zeitbedraf für eine Probe; abhängig von der verwendeten Methode ist der Gesamtzeitbedarf für mehrere Proben meist weniger als ein Vielfaches dieser Zeitangabe.

**der Coulter counter liefert das Netto-Biomasse-Volumen, wohingegen Particle Tracking das Brutto-Volumen liefert;

***hier ist die rote Farbe der diskriminierende Faktor, aber auch andere Farben oder Graustufen (dunklere, dichtere Granulen im Vergleich zu Belebtschlamm) können als Faktor herangezogen werden und das Protokoll entsprechend abgewandelt werden;

****die erste Zeit beinhaltet ebenso die Bildung der Standards und die DNA-Extraktion, die zweite nur den qPCR Lauf;

Tabelle 5: Korrelation der AC/AnAOB Quantifizierungsergebnisse zwischen allen untersuchten Methoden für alle sechs Probentypen (beide Probenmatrizzen); Die Pearson's Korrelationskoeffizienten sind gemeinsam mit den jeweiligen Signifikanzlevels angegeben (Exponent); Exponenten definiert wie folgt: a (p<0.01), b (p<0.001) and c (p<0.0005);

|  | GA | PT sieved | PT total | HQ | qPCR | AM |
|---|---|---|---|---|---|---|
| GA | - | 0.95[c] | 0.94[c] | 0.71[c] | 0.54[b] | 0.37[b] |
| PT sieved | 0.95[c] | - | 0.99[c] | 0.78[c] | 0.69[c] | 0.49[c] |
| PT total | 0.94[c] | 0.99[c] | - | 0.78[c] | 0.68[c] | 0.53[c] |
| HQ | 0.71[c] | 0.78[c] | 0.78[c] | - | 0.81[1] | 0.30[a] |
| qPCR | 0.54[b] | 0.69[c] | 0.68[c] | 0.81[c] | - | 0.38[c] |
| AM | 0.37[b] | 0.49[c] | 0.53[c] | 0.30[a] | 0.38[c] | - |

Tabelle 6: Korrelation der AC/AnAOB Quantifizierungsergebnisse zwischen allen untersuchten Methoden für die SL-Probenmatrix; Die Pearson's Korrelationskoeffizienten sind gemeinsam mit den jeweiligen Signifikanzlevels angegeben (Exponent); Exponenten definiert wie folgt: a (p<0.01), b (p<0.001) and c (p<0.0005);

|  | GA | PT sieved | PT total | HQ | qPCR | AM | CC |
|---|---|---|---|---|---|---|---|
| GA | - | 0.99[c] | 0.99[c] | 0.99[c] | 0.75[b] | 0.99[c] | 0.96[c] |
| PT sieved | 0.99[c] | - | 1[c] | 1[c] | 0.73[b] | 0.99[c] | 0.98[c] |
| PT total | 0.99[c] | 1[c] | - | 0.99[c] | 0.73[b] | 0.99[c] | 0.98[c] |
| HQ | 0.99[c] | 1[c] | 0.99[c] | - | 0.71[b] | 0.99[c] | 0.97[c] |
| qPCR | 0.75[b] | 0.73[b] | 0.73[b] | 0.71[b] | - | 0.75[b] | 0.72[b] |
| AM | 0.99[c] | 0.99[c] | 0.99[c] | 0.99 | 0.75[b] | - | 0.96[c] |
| CC | 0.96[c] | 0.98[c] | 0.98[c] | 0.97[c] | 0.72[b] | 0.96[c] | - |

Tabelle 7: Korrelation der AC/AnAOB Quantifizierungsergebnisse zwischen allen untersuchten Methoden für die B-Probenmatrix; Die Pearson's Korrelationskoeffizienten sind gemeinsam mit den jeweiligen Signifikanzlevels angegeben (Exponent); Exponenten definiert wie folgt: a (p<0.05), b (p<0.001) and c (p<0.0005);

|  | GA | PT sieved | PT total | HQ | qPCR | AM |
|---|---|---|---|---|---|---|
| GA | - | 0.99[b] | 0.97[b] | 0.99[b] | 0.44[a] | -0.15 |
| PT sieved | 0.99[b] | - | 1[c] | 0.96[a] | 0.47[a] | -0.12 |
| PT total | 0.97[b] | 1[c] | - | 0.94[a] | 0.45[a] | -0.06 |
| HQ | 0.99[b] | 0.96[a] | 0.94[a] | - | 0.41[a] | -0.17 |
| qPCR | 0.44[a] | 0.47[a] | 0.45[a] | 0.41[a] | - | -0.20 |
| AM | -0.15 | -0.12 | -0.06 | -0.17 | -0.20 | - |

**Gravimetrische Analyse von Anammox-Granulae (GA)**

Wenn Anammox-Granulae in hoher Dichte vorliegen, die Biomasse nicht zu stark schleimig und der Anteil an partikulären Störstoffen im Verhältnis gering sind, kann diese einfache Bestimmung (absieben, waschen, trocknen, wiegen) für ein Monitoring genützt werden. Je nach Größenverteilung der Granulae und Störstoffe kann die Maschenweite des Siebes angepasst werden. Im hier untersuchten Fall konnten mit einem 0,125 mm-Sieb im DEMON®-Becken (SL-Proben) die verschiedenen Fraktionen korrekt unterschieden und mit der Anammox-Aktivität korreliert werden.

**Quantifizierung des Häm-Gehaltes (HQ)**

Häme agieren als prosthetische Gruppen vieler Enzyme in allen Lebensformen (Gumiero et al. 2011). Besonders hohe Häm-Gehalte führen zu einer Rotfärbung der Zellen, wie bei den roten Blutzellen und den Anammox-Bakterien. In letzteren sind sie essentieller Bestandteil des Stoffwechselwegs der anaeroben Ammoniumoxidation, dessen Enzyme bis zu 20% des gesamten Proteingehalts dieser Zellen ausmachen können (Jetten et al., 2009), während der Häm-Gehalt in anderen Mikroorganismen demgegenüber fast vernachlässigbar ist. Damit ist der Häm-Gehalt einer Kläranlagen-Biomasse ein spezifischer Parameter zur indirekten Bestimmung des Gehalts an Anammox-Bakterien bzw. deren relevanter Enzyme. Die Bestimmung von Häm ist für die Analyse medizinischer Proben wie zum Beispiel Faeces oder Gewebe länger bekannt (Sinclair et al. 2001), musste aber für die Anwendung an Bakterien optimiert werden.

Ein Zusammenhang zwischen optisch sichtbarer Rotfärbung der Anammox-Biomasse und ihrer Stoffwechselaktivität wurde schon mehrfach beobachtet (eigene Untersuchungen und (Ali et al. 2013), insbesondere, dass sowohl sehr helle als auch dunkel bis schwarz gefärbte Granulae eine deutlich verringerte Leistung zeigen. Die Analyse des Hämgehalts der Biomasse ist in Kombination mit optischen Methoden (PT) eine einfache aber sichere Methode zum Monitoring von Abundanz und Vitalität der Anammoxpopulation vor Ort.

## Zählung und Vermessung der Granulae mittels Coulter Counter (CC) und Bildanalyse (PT)

Diese beiden Methoden unterscheiden sich von allen anderen dadurch, dass neben den Quantifizierungsparametern für Anammox-Biomasse, das sind Granulenzahl (CC und PT), gesamt-Granulenquerschnittsfläche (PT) und gesamt-Granulenvolumen (CC und PT), auch Größenverteilungen der Partikel erfasst werden. Diese Information ist z.B. relevant, wenn Biomassewachstum und -zusammensetzung bewertet (Nielsen et al. 2005, Volcke et al. 2010, Winkler et al. 2011) oder die Wirkungsweise der Hydrozyklone im Detail untersucht werden soll.

## Quantitative PCR (qPCR)

Dass alle bisher bekannten Anammox-Bakterien derselben monophyletischen Ordnung *Planctomycetales* angehören, erleichtert deren molekularbiologische Quantifizierung über spezifische 16S rRNA-Primer (Kuenen 2008, Li and Gu 2011). Die Methode bietet einerseits die höchstmögliche Spezifität und daher grundsätzlich höchste Richtigkeit für die Biomassebestimmung, beinhaltet aber neben dem hohen Aufwand auch einige potentielle Fehlerquellen. Insbesondere musste hier das Standardprotokoll zur DNA-Extraktion aus Gram-positiven Bakterienzellen deutlich modifiziert und um mechanische Zelllyseschritte ergänzt werden, um eine ausreichende, stabile DNA-Ausbeute aus der granulären Biomasse zu erzielen.

## Physiologische Aktivitätsmessung (AM)

Die Umsatzraten der wesentlichen gelösten Stickstoffspezies Ammonium, Nitrit und Nitrat werden häufig zur Bewertung der physiologischen Aktivität von Anammoxbakterien, unabhängig von ihrer Wuchsform, herangezogen. Hier konnte gezeigt werden, dass diese Methode sehr gut für Klärschlämme mit hohem Anammoxgehalt, wie im DEMON®-Reaktor üblich, geeignet ist. In Proben mit hohem Anteil anderer Mikroorganismen, wie im untersuchten, Anammox-angereicherten Hauptstrombecken, sind die Messungen wegen der Überlagerung durch andere Stoffwechselprozesse oft schwer zu interpretieren. Insbesondere Denitrifikation und Ammonifikation (Wett et al. 2013) stören hier am meisten, führen zu oftmals starker Unterschätzung der Anammoxaktivität und konnten bisher noch nicht ausreichend selektiv gehemmt werden. Vor allem im Zyklon-Oberlauf ist das Verhältnis von abgereicherter Anammox-Biomasse zu anderer Biomasse sehr ungünstig und macht eine Aktivitätsmessung praktisch unmöglich.

## Schlussfolgerungen

Mit allen Methoden, die hier zur Abschätzung von Anammox-Biomasse oder deren Stickstoff-Umsatzleistung untersucht wurden, konnten die drei verschiedenen Proben aus dem DEMON®-Reaktor (Becken, Zyklon-Ober- und -unterlauf) korrekt unterschieden und gereiht werden. Für die Untersuchung der Proben aus beiden Reaktoren (DEMON® und Hauptstrom) waren Hämbestimmung und quantitative PCR am besten geeignet.

Mit den materiell aufwändigeren und Forschungslabors vorbehaltenen Methoden qPCR und Coulter Counter konnten Ergebnisse und Zuverlässigkeit der weniger anspruchsvollen, in jedem Routinelabor durchführbaren Techniken Particle Tracking und Hämbestimmung bestätigt werden. Grundsätzlich korrelierten alle Methoden positiv, insbesondere ergab die Probe mit dem höchsten Anammoxgehalt bei allen Methoden die höchsten Werte. Zusammenfassend wird folgende Monitoringstrategie für Kläranlagen mit Anammox-Technologie vorgeschlagen: Regelmäßige Häm-Bestimmung und Particle Tracking ergänzen sich ideal zur Abundanz- und Vitalitätskontrolle der Anammox-Bakterien. Gelegentlich sollten auch Aktivitätstests durchgeführt werden.

## Optimierung der Häm-Extraktion und Bestimmung aus Anammox-Bakterien

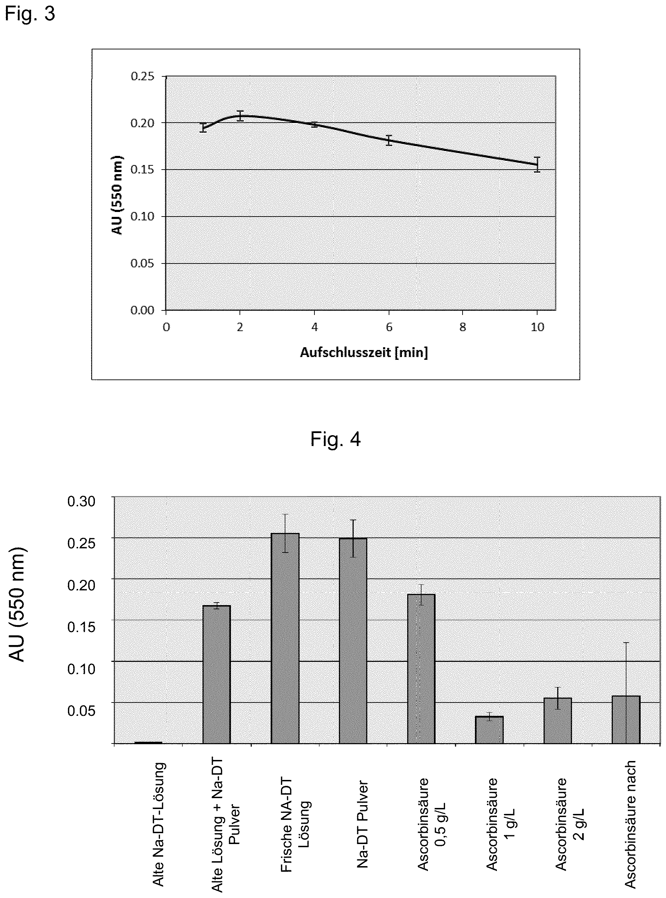

Die Optimierung der Hämextraktion wurde mit Anammox-Biomasse aus dem DEMON®-Reaktor Strass im Zillertal durchgeführt und an mehreren anderen Anlagen getestet. Aus den Testreihen zu Laugenstärke (Fig. 2) und Aufschlusszeit (Fig. 3) wurde die optimale Kombination von Aufschlussgrad und Zeitunempfindlichkeit mit einer NaOH-Konzentration von 1 mol/L und einer Aufschlusszeit von 2 min bei 100°C in einem 1,5 mL-Mikroreaktionsgefäß in kochendem Wasser ermittelt. Für abweichende Volumina bzw. Heiztechniken (z.B. Thermoblock) ist die optimale Aufschlusszeit neu zu bestimmen.

Fig. 4 zeigt die Bedeutung der Zugabe frisch zubereiteter Natriumdithionit-Lösung (alt = älter als 24 h; frisch = jünger als 2h). Ascorbinsäure ist als Reduktionsmittel nicht geeignet. Anstelle einer NaDT-Lösung in 1 M NaOH kann das Reagens auch als Pulver zugegeben werden (Fig. 4-5).

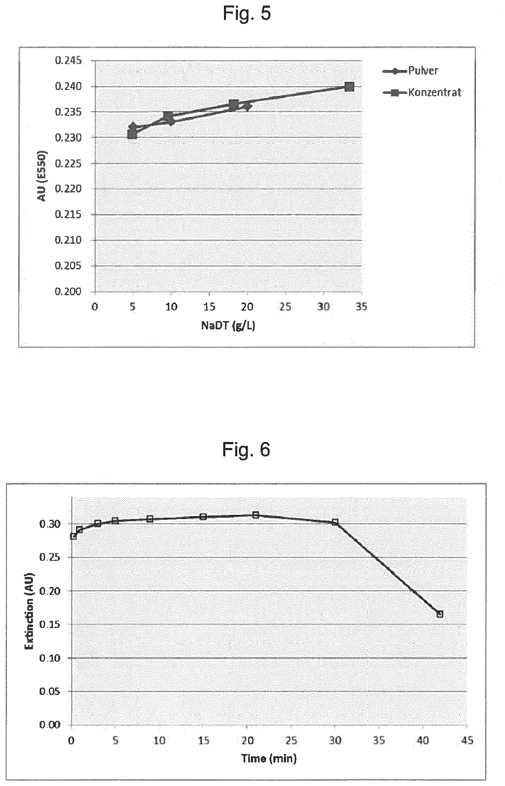

Der rote Farbstoff bleibt nach Extraktion und Reduktion für etwa 20 min stabil und kann in dieser Zeit ohne signifikante Veränderung photometrisch gemessen werden (Fig. 6). Der Peak des reduzierten Hämochroms bei 550 nm lässt sich optimal durch eine Messung bei 3 Wellenlängen bestimmen. Durch die Messpunkte bei 535 und 570 nm wird die Basislinie konstruiert und die Peakhöhe bei 550 nm darauf bezogen (3-Wellenlängen-Kalibration jedes modernen Photometers).

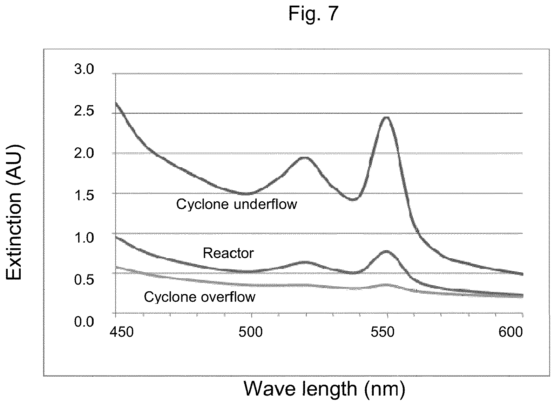

Fig. 7 zeigt beispielhaft die Ergebnisse aus Proben des Zyklon-Zulaufs (= Reaktor), - Unterlaufs und -Oberlaufs des DEMON®-Reaktors der Kläranlage Tobl/Bruneck, (Zweck des Hydrozyklons ist es, die Anammox-Biomasse via Zyklon-Unterlauf im Reaktor zu halten und den Überschuss an weniger dichten, schnellwüchsigeren Nitrifikanten- und heterotrophen Bakterien-Biomasse via Zyklon-Oberlauf aus dem Reaktor zu entnehmen.)

Ein Beispiel der Durchführung sah z.B. so aus:

Schritt 1: Probenahme
Schritt 2: Abfüllung von ca. 8-10 mL der Probe in ein Röhrchen aus dem Kit, in dem trocken ca. 200 mg Natrium-Dithionit vorgelegt sind
Schritt 3: Zugabe von ca. 1 mL Natronlauge aus dem Kit (Konzentration ca. 10-11 mol/L), sodass die Endkonzentration in der Mischung 1 mol/L beträgt
Schritt 4: Erhitzen der verschlossenen Röhrchen auf 70° für 10 min
Schritt 5: Abkühlen lassen auf Raumtemperatur
Schritt 6: Filtrieren und Mischen in leeren Küvetten (im Kit Rundküvetten aus Glas wie bei anderen Nanocolor-Tests)
Schritt 7: Filtrat im Spektrophotometer messen.

## Referenzen

Ali, M., Chai, L.Y., Tang, C.J., Zheng, P., Min, X.B., Yang, Z.H., Xiong, L. and Song, Y.X. (2013) The increasing interest of ANAMMOX research in China: bacteria, process development, and application. Biomed Res Int 2013, 134914.

Coulter, W., H. (1956) High Speed Automatic Blood Cell Counter and Cell Size Analyses. Proceedings of the National Electronics Conference 112, 10341040.

Gumiero, A., Metcalfe, C.L., Pearson, A.R., Raven, E.L., Moody, P.C.E. (2011) Nature of Ferryl Heme in Compounds I and II. J Biol Chem 286(20), 1260-1268.

Hammer, O., Harper, D. and Ryan, P. (2001) Paleontological Statistics Software Package for Education and Data Analysis. Palaeontologia Electronica 4, 1-9.

Jetten, M.S., Wagner, M., Fuerst, J., van Loosdrecht, M., Kuenen, G. and Strous, M. (2001) Microbiology and application of the anaerobic ammonium oxidation ('anammox') process. Curr Opin Biotechnol 12(3), 283-288.

Jetten, M., Van Niftrik, L. and Strous, M. (2009) Biochemistry and molecular biology of anammox bacteria. Critical Reviews in Biochemistry and Molecular Biology 44, 65-84.

Kuenen, J.G. (2008) Anammox bacteria: from discovery to application. Nature Reviews Microbiology 6(4), 320-326.

Li, M. and Gu, J.D. (2011) Advances in methods for detection of anaerobic ammonium oxidizing (anammox) bacteria. Appl Microbiol Biotechnol 90(4), 1241-1252.

Mantel, N. (1967) The detection of disease clustering and a generalized regression approach. Cancer Res 27(2), 209-220.

Mantel, N. and Valand, R.S. (1970) A technique of nonparametric multivariate analysis. Biometrics 26(3), 547-558.

Nielsen, M., Bollmann, A., Sliekers, O., Jetten, M., Schmid, M., Strous, M., Schmidt, I., Larsen, L.H., Nielsen, L.P. and Revsbech, N.P. (2005) Kinetics, diffusional limitation and microscale distribution of chemistry and organisms in a CANON reactor. FEMS Microbiol Ecol 51(2), 247-256.

Sinclair, P.R., Gorman, N. and Jacobs, J.M. (2001) Measurement of heme concentration. Curr Protoc Toxicol Chapter 8, Unit 8 3.

Volcke, E.I., Picioreanu, C., Baets, B.D. and Loosdrecht, M.C. (2012) The granule size distribution in an anammox-based granular sludge reactor affects the conversion - implications for modelling. Biotechnol Bioeng.

Volcke, E.I.P., Picioreanu, C., De Baets, B. and van Loosdrecht, M.C.M. (2010) Effect of granule size on autotrophic nitrogen removal in a granular sludge reactor. Environmental Technology 31(11), 1271-1280.

Wett, B., Omari, A., Podmirseg, S.M., Han, M., Akintayo, O., Gomez Brandon, M., Murthy, S., Bott, C., Hell, M., Takacs, I., Nyhuis, G. and O'Shaughnessy, M. (2013) Going for mainstream deammonification from bench to full scale for maximized resource efficiency. Water Sci Technol 68(2), 283-289.

Winkler, M.K.H., Kleerebezem, R., Kuenen, J.G., Yang, J.J. and van Loosdrecht, M.C.M. (2011) Segregation of Biomass in Cyclic Anaerobic/Aerobic Granular Sludge Allows the Enrichment of Anaerobic Ammonium Oxidizing Bacteria at Low Temperatures. Environmental Science & Technology 45(17), 7330-7337.

## Patentansprüche

1. Verfahren zur Ermittlung der Menge an Anammox-Bakterien ohne Häm-Derivatisierung mit Pyridin und Oxidierung von Häm und anschließender vergleichender Messung von oxidiertem und reduziertem Häm in einem Bioreaktor, umfassend die Schritte

   i) Entnehmen einer Probe aus dem Bioreaktor;
   ii) gegebenenfalls Abtrennen der festen Bestandteile von den flüssigen Bestandteilen, bei Vorhandensein von rotgefärbten Verunreinigungen im Wellenlängenbereich 500 nm bis 600 nm, wobei der Überstand verworfen wird;
   iii) Versetzen der Probe mit Metallhydroxid;
   iv) gegebenenfalls Suspendieren der festen Bestandteile in der alkalischen Lösung;
   v) Erwärmen der Proben auf zumindest 60°C;
   vi) Abtrennen fester Bestandteile;
   vii) Zugabe eines Salz der dithionigen Säure als Reduktionsmittel zur flüssigen Phase;
   viii) Messen der Lichtdurchlässigkeit der flüssigen Phase in einem Spektrophotometer bei drei Wellenlängen im Bereich 500 bis 600 nm, wobei die Wellenlängenmessung der ersten Wellenlänge bei 530 bis 540 nm, vorzugsweise 535 nm, der zweiten Wellenlänge bei 545 bis 555 nm, vorzugsweise bei 550 nm und der dritten Wellenlänge bei 565 bis 575, vorzugsweise bei 570 nm erfolgt;
   ix) Vergleich der gemessenen Lichtdurchlässigkeit mit einem Referenzspektrum eines Häm-Proteins mit bekanntem Häm-Gehalt.

2. Verfahren nach Anspruch 1, **dadurch gekennzeichnet, dass** das Abtrennen der festen Bestandteile mittel Zentrifugation erfolgt.

3. Verfahren nach Anspruch 1 oder Anspruch 2, **dadurch gekennzeichnet, dass** das Salz der dithionigen Säure $Na_2S_2O_4$ ist.

4. Verfahren nach einem der Ansprüche 1 bis 3, **dadurch gekennzeichnet, dass** der Bioreaktor ein Klärbecken ist.

## Claims

1. A method for determining the amount of Anammox bacteria without heme derivatization with pyridine and oxidation of heme and subsequent comparative measuring of oxidized and reduced heme in a biochemical reactor, comprising the following steps:

   i) taking a sample of the biochemical reactor;
   ii) optionally, separating the solid components from the liquid components, upon presence of red-stained contaminations in the wave range of 500 nm to 600 nm, with the supernatant being discarded;
   iii) adding metal hydroxide to the sample;
   iv) optionally suspending the solid components in the alkaline solution;
   v) heating the samples up to at least 60°C;
   vi) separating the solid components;
   vii) adding a salt of the dithionic acid as a reducing agent to the liquid phase;
   viii) measuring the translucency of the liquid phase in a spectrophotometer at three wave lengths in the range of 500 to 600 nm, wherein the wave length measurement of the first wave length is carried out at 530 to 540 nm, preferably 535 nm, of the second wave length at 545 to 555 nm, preferably at 550 nm, and of the third wave length at 565 to 575 nm, preferably at 570 nm;
ix) comparing the translucency measured with a reference spectrum of a heme protein having a heme content that is known.

2.  A method according to claim 1, **characterized in that** the separation of the solid components is carried out by centrifugation.

3.  A method according to claim 1 or claim 2, **characterized in that** the salt of the dithionic acid is $Na_2S_2O_4$.

4.  A method according to any of claims 1 to 3, **characterized in that** the biochemical reactor is a clarifier basin.


**Revendications**

1.  Procédé de détermination de la quantité de bactéries anamox sans dérivation de hème avec de la pyridine et oxydation de hème et mesure comparative consécutive de hème oxydée et réduite dans un bioréacteur, comprenant les étapes suivantes:

    i) prélever un échantillon hors du bioréacteur;
    ii) éventuellement séparer les composants solides des composants liquides, en présence d'impuretés colorées en rouge dans le domaine des longueurs d'onde de 500 nm à 600 nm, l'excédent étant rejeté;
    iii) mélanger l'échantillon avec de l'hydroxyde métallique;
    iv) éventuellement mettre les composants solides en suspension dans la solution alcaline;
    v) chauffer les échantillons à au moins 60°C;
    vi) séparer les composants solides;
    vii) ajouter un sel de l'acide dithionique comme agent réducteur à la phase liquide;
    viii) mesurer la transparence de la phase liquide dans un spectrophotomètre à trois longueurs d'onde dans le domaine de 500 à 600 nm, dans lequel on effectue la mesure de longueur d'onde de la première longueur d'onde de 530 à 540 nm, de préférence à 535 nm, de la deuxième longueur d'onde de 545 à 555 nm, de préférence à 550 nm, et de la troisième longueur d'onde de 565 à 575 nm, de préférence à 570 nm; et
    ix) comparer la transparence mesurée avec un spectre de référence d'une protéine à hème avec une teneur en hème connue.

2.  Procédé selon la revendication 1, **caractérisé en ce que** l'on effectue la séparation des composants solides par centrifugation.

3.  Procédé selon la revendication 1 ou la revendication 2, **caractérisé en ce que** le sel de l'acide dithionique est le $Na_2S_2O_4$.

4.  Procédé selon l'une quelconque des revendications 1 à 3, **caractérisé en ce que** le bioréacteur est un bassin de décantation.

## Fig. 1

## Fig. 2

Fig. 3

Fig. 4

## Fig. 5

## Fig. 6

Fig. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2007033393 A1

### In der Beschreibung aufgeführte Nicht-Patentliteratur

- **SINCLAIR et al.** Curr Protoc Toxicol. Mai 2001
- **ALI, M. ; CHAI, L.Y. ; TANG, C.J. ; ZHENG, P. ; MIN, X.B. ; YANG, Z.H. ; XIONG, L. ; SONG, Y.X.** The increasing interest of ANAMMOX research in China: bacteria, process development, and application. *Biomed Res Int,* 2013, 134914
- **COULTER, W., H.** High Speed Automatic Blood Cell Counter and Cell Size Analyses. *Proceedings of the National Electronics Conference,* 1956, vol. 112, 10341040
- **GUMIERO, A. ; METCALFE, C.L. ; PEARSON, A.R. ; RAVEN, E.L. ; MOODY, P.C.E.** Nature of Ferryl Heme in Compounds I and II. *J Biol Chem,* 2011, vol. 286 (20), 1260-1268
- **HAMMER, O. ; HARPER, D. ; RYAN, P.** Paleontological Statistics Software Package for Education and Data Analysis. *Palaeontologia Electronica,* 2001, vol. 4, 1-9
- **JETTEN, M.S. ; WAGNER, M. ; FUERST, J. ; VAN LOOSDRECHT, M. ; KUENEN, G. ; STROUS, M.** Microbiology and application of the anaerobic ammonium oxidation ('anammox') process. *Curr Opin Biotechnol,* 2001, vol. 12 (3), 283-288
- **JETTEN, M. ; VAN NIFTRIK, L. ; STROUS, M.** Biochemistry and molecular biology of anammox bacteria. *Critical Reviews in Biochemistry and Molecular Biology,* 2009, vol. 44, 65-84
- **KUENEN, J.G.** Anammox bacteria: from discovery to application. *Nature Reviews Microbiology,* 2008, vol. 6 (4), 320-326
- **LI, M. ; GU, J.D.** Advances in methods for detection of anaerobic ammonium oxidizing (anammox) bacteria. *Appl Microbiol Biotechnol,* 2011, vol. 90 (4), 1241-1252
- **MANTEL, N.** The detection of disease clustering and a generalized regression approach. *Cancer Res,* 1967, vol. 27 (2), 209-220
- **MANTEL, N. ; VALAND, R.S.** A technique of nonparametric multivariate analysis. *Biometrics,* 1970, vol. 26 (3), 547-558
- **NIELSEN, M. ; BOLLMANN, A. ; SLIEKERS, O. ; JETTEN, M. ; SCHMID, M. ; STROUS, M. ; SCHMIDT, I. ; LARSEN, L.H. ; NIELSEN, L.P. ; REVSBECH, N.P.** Kinetics, diffusional limitation and microscale distribution of chemistry and organisms in a CANON reactor. *FEMS Microbiol Ecol,* 2005, vol. 51 (2), 247-256
- Measurement of heme concentration. **SINCLAIR, P.R. ; GORMAN, N. ; JACOBS, J.M.** Curr Protoc Toxicol. 2001
- **VOLCKE, E.I. ; PICIOREANU, C. ; BAETS, B.D. ; LOOSDRECHT, M.C.** The granule size distribution in an anammox-based granular sludge reactor affects the conversion - implications for modelling. *Biotechnol Bioeng,* 2012
- **VOLCKE, E.I.P. ; PICIOREANU, C. ; DE BAETS, B. ; VAN LOOSDRECHT, M.C.M.** Effect of granule size on autotrophic nitrogen removal in a granular sludge reactor. *Environmental Technology,* 2010, vol. 31 (11), 1271-1280
- **WETT, B. ; OMARI, A. ; PODMIRSEG, S.M. ; HAN, M. ; AKINTAYO, O. ; GOMEZ BRANDON, M. ; MURTHY, S. ; BOTT, C. ; HELL, M. ; TAKACS, I.** Going for mainstream deammonification from bench to full scale for maximized resource efficiency. *Water Sci Technol,* 2013, vol. 68 (2), 283-289
- **WINKLER, M.K.H. ; KLEEREBEZEM, R. ; KUENEN, J.G. ; YANG, J.J. ; VAN LOOSDRECHT, M.C.M.** Segregation of Biomass in Cyclic Anaerobic/Aerobic Granular Sludge Allows the Enrichment of Anaerobic Ammonium Oxidizing Bacteria at Low Temperatures. *Environmental Science & Technology,* 2011, vol. 45 (17), 7330-7337